# DEMANDE DE BREVET EUROPEEN

(11) **EP 0 658 309 A1**
(43) Date de publication de la demande: **21.06.1995**
(21) Numéro de dépôt: 94402865.3
(22) Date de dépôt: 13.12.1994
(51) Int. Cl.: A01N 59/00, A01N 37/16, A61L 2/18, A61L 11/00

(54) **Composition liquide désinfectante et ses utilisations**

(30) Priorité: 14.12.1993 FR 9314981
(71) Demandeur: CHEMOXAL S.A., F-75007 Paris (FR)
(72) Inventeur: Crisinel, Pascal, F-78000 Versailles (FR); Le Rouzic, Daniel, F-95120 Ermont (FR)
(74) Mandataire: Caen, Thierry Alain

(57) **Abrégé**

Une composition liquide désinfectante comportant au moins un peroxyacide désinfectant et au moins un agent colorant, ladite composition étant stable pendant plus de quinze jours. Cette composition peut être utilisée pour la désinfection et la coloration des eaux, d'un article ou d'une surface.

## Description

La présente invention concerne une composition liquide désinfectante colorée et stable dans le temps, ainsi que ses utilisations, notamment pour la désinfection d'articles, tels des déchets hospitaliers, d'une surface, en particulier d'une surface d'un corps humain ou animal.

De nombreux agents désinfectants, tels par exemple, le dioxyde de chlore, le dodécylbenzène sulfonate, la chlorhexidine, ou l'hypochlorite de sodium, sont commercialisés sous forme de solutions colorées. La coloration de ces solutions permet notamment de les différencier d'autres produits. Ces compositions désinfectantes sont destinées à être appliquées sur une surface d'un corps humain ou animal, généralement la peau ou les muqueuses, par exemple, en vue de la désinfection des trayons bovins.

Par ailleurs, il est bien connu que les peroxyacides organiques sont des agents désinfectants efficaces. Toutefois, il n'a pas été possible jusqu'à présent de mettre au point des compositions liquides colorées à base de tels peroxydes, stables dans le temps. Ainsi, H. MÜCKE, "Über Möglichkeiten Anfärbung von Peressigsäure für Hautesinfektion", Pharmazie, 29, H.3 (1974), pages 206-207, a décrit des compositions à base d'acide péracétique colorées par des agents colorants tels l'éosine, le vert de méthylène, le bleu de méthylène, le méthyle violet, la sulforhodamine B ou le vert malachite. Cependant, de telles compositions ne sont stables qu'un seul jour. L'utilisation de telles compositions nécessite donc de les préparer dans les heures précédant leurs utilisations. Leur stockage est donc impossible.

Un objet de la présente invention consiste donc en des compositions liquides désinfectantes colorées à base de peroxydes, stables dans le temps. Ces compositions ne nécessitent pas d'être préparées dans les heures précédant leur utilisation et peuvent être stockées pendant plusieurs mois, voire plusieurs années.

Un autre objet de l'invention consiste en l'utilisation de compositions désinfectantes colorées à base de peroxyde, notamment pour la désinfection et la coloration des eaux, d'articles ou de surfaces.

Un troisième objet de l'invention consiste en l'utilisation d'une solution aqueuse désinfectante colorée pour la désinfection et le marquage d'un déchet.

L'invention consiste ainsi en une composition liquide désinfectante caractérisée en ce qu'elle comporte au moins un peroxyacide désinfectant et au moins un agent colorant, ledit peroxyacide organique étant choisi dans le groupe constitué par
1) un monoperoxyde de formule (I) :

   R₁ - CO₃H (I)

   dans laquelle R₁ représente un radical alkyle en C₁-C₂₄, linéaire ou ramifié, un radical aryle ou un radical cycloalkyle en C₃-C₁₀
2) un diperoxyde de formule (II) :

   H₃OC - R₂ - CO₃H (II)

   dans laquelle R₂ représente un radical alkyle en C₁-C₂₄, linéaire ou ramifié, un radical arylène ou un radical cycloalkylène en C₃-C₁₀,

chacun de R₁ ou R₂ étant substitué ou non par un ou plusieurs groupes fonctionnels ou radicaux, ladite composition étant stable pendant plus de quinze jours.

Habituellement, les compositions selon l'invention peuvent être stables pendant une période nettement supérieure par exemple de l'ordre de 6 mois à 1 an.

Par le terme "stable", on entend ici que le titre en peroxyacide de la composition liquide ainsi que sa coloration, plus particulièrement son intensité colorométrique, présentent une variation inférieure à 10 % sur une période de temps donné.

Dans le cadre de la présente invention, et sauf indication contraire, lorsqu'un radical est substitué, les substituants sont de préférence choisis dans le groupe constitué par les groupes fonctionnels ou radicaux suivants :
- un groupe carboxylique libre sous forme esters ou amides ou salifiés par un contre-ion alcalin, alcalino-terreux, ammonium ou phosphonium,
- un radical alkyle en C₁ - C₂₄, linéaire ou ramifié, substitué ou non par une ou plusieurs, généralement par une à cinq fonctions carboxyliques, et/ou amides,
- un radical acyle de formule :

   R-CO-,

   R représentant un radical alkyle en C₁ - C₂₄, linéaire ou ramifié,
- un radical alkoxy en C₁ - C₂₄, substitué ou non par une ou plusieurs, généralement par une à cinq, fonctions carboxyliques et/ou amines,
- un radical aryle,
- une fonction hydroxyle, nitrile, trifluorométhyle, sulfonyle,
- un atome d'halogène tel le fluor, le chlore, ou le brome,

A titre de monoperoxyacide de formule (I) préféré, on peut citer ceux où R₁ représente un radical alkyle en C₁ - C₁₂.

Parmi les peroxyacides de formule (I) ou (II) on peut notamment citer les acides peracétique, perpropionique, monoperoxy-azélaïque, percaprylique, perundécylénique, perlaurique, monoperoxysuccinique, monoperoxygluratique, diperoxysuccinique, diperoxyazélaïque, octyl-2-peroxy-butane-dioïque, 1-4, décyl-2-peroxybutanedioïque,4, dodécyl-2-peroxybutanedioïque, 1-4, diperoxydodécanedioïque, 1-2, l'acide peracétique étant un peroxyacide organique particulièrement préféré dans le cadre de la présente invention. On peut également citer l'acide peroxybenzoïque et ses dérivés tels les acides métachloroperoxybenzoïque, p-tertio-butylperoxybenzoïque, p-nitroperoxybenzoïque, mono-perphtalique ou amidophtaloylpéracétique ou amidophtaloylhexanoïque.

La teneur en peroxyacide de la composition selon l'invention peut être comprise entre 0,0001% et 5%, de préférence entre 0,001 % et 1 %, plus préférentiellement entre 0,001% et 0,4 %. Ces teneurs, ainsi que, sauf indication contraire, toute teneur indiquée ci-dessous, sont à considérer pour une composition ayant atteint l'équilibre.

Les agents colorants pouvant être mis en oeuvre dans la composition selon l'invention peuvent être tout agent colorant permettant de maintenir la composition selon l'invention stable pendant au moins quinze jours et de préférence pendant 6 à 12 mois. Avantageusement, ces agents colorants sont de qualité alimentaire, selon les normes définies par la législation de la Communauté européenne pour ce qui concerne les additifs alimentaires.

Un agent colorant préféré selon l'invention consiste en un colorant du type monoazo, c'est-à-dire un composé comportant une fonction azo - N = N -.

Ces composés sont des colorants bien connus. Ils sont par exemple décrits dans "Nomenclature of Organics Chemistry" -mentionné ci-dessus-, pages 277 à 283.

Plus particulièrement, l'agent colorant peut être un composé monoazo de formule (III) :

R₃ - N = N - R₄ (III)

dans laquelle R₃ et R₄, identiques ou différents, représentent un radical aryle, de préférence un radical naphtyle ou un radical phényle, chacun de R₃ et R₄ étant substitué ou non par un à trois groupes fonctionnels OH ou -SO₃M, M étant l'hydrogène ou un cation d'un métal alcalin tel le sodium. De tels composés sont par exemple commercialisés par la société Wackherr sous les désignations rouge vif W 3002 et orange soleil W 2002.

D'autres agents colorants préférés selon l'invention consistent en les pigments. Les pigments sont des composés bien connus de nature organique, inorganique ou organométalliques. Contrairement aux colorants, les pigments sont des agents colorants insolubles dans l'eau, dans laquelle ils forment des dispersions. Des pigments tout particulièrement préférés consistent en les phtalocyanines.

En vue d'améliorer leur dispersion en solution, les pigments selon l'invention peuvent se présenter sous la forme d'une dispersion de pigments prête à l'emploi comprenant, outre le pigment proprement dit, des agents dispersants et des agents mouillants non ioniques. Des dispersions prêtes à l'emploi de ce type comprenant des phtalocyanines sont commercialisées par la société Hoechst, sous la marque "HOSTAFINE".

Généralement, la composition selon l'invention comporte de 0,0001% à 1%, de préférence de 0,001% à 0,5% en poids en ledit agent colorant.

Selon un aspect particulier de l'invention, le ou les agents colorants et peroxyacides sont en solution dans un solvant, par exemple un solvant organique, de préférence autre qu'un mono-alcool, mais tout préférentiellement un tel solvant consiste en l'eau.

Généralement, ladite composition comporte également un acide carboxylique. Avantageusement, lorsque le peroxyacide organique est un composé de formule (I) ou un composé de formule (II) tels que définis ci-dessus, l'acide carboxylique est choisi parmi, respectivement, un composé de formule (IV)

R₁COOH (IV)

ou de formule (V)

HOOC-R₂-COOH (V)

R₁ et R₂ ayant la même signification que celle dudit peroxyacide organique.

Lorsque le peroxyacide organique est l'acide peracétique, l'acide carboxylique présent dans la composition selon l'invention est donc l'acide acétique. La teneur en acide carboxylique dans la composition selon l'invention peut être comprise entre 0,01 et 20 % en poids.

En outre, une composition selon l'invention peut comporter du peroxyde d'hydrogène. La concentration en celui-ci dans la composition peut être comprise entre 0,001 et 35 %, de préférence entre 0,1 et 10 % en poids. Le peroxyde d'hydrogène mis en oeuvre se présente de préférence sous forme purifiée, c'est-à-dire substantiellement débarrassé de ses impuretés organiques et métalliques. Le peroxyde d'hydrogène purifié peut être obtenu selon des procédés connus de l'homme du métier, notamment par distillation.

Le pH d'une composition liquide selon l'invention est habituellement inférieur à 5, de préférence compris entre 1 et 3,5.

Une composition liquide désinfectante préférée dans le cadre de la présente invention consiste en une solution aqueuse comprenant de 0,1 à 10 % en poids de peroxyde d'hydrogène, de 0,001% à 0,4% en poids d'un peroxyacide organique tels ceux définis ci-dessus, et de 0,1 % à 10 % en poids d'un acide carboxylique, de 0,0001% à 0,03% en poids d'un agent colorant consistant en un composé azo ou un pigment tels ceux définis ci-dessus.

Outre les composés mentionnés ci-dessus, la composition selon l'invention peut également comporter des tensioactifs. Ceux-ci peuvent être des tensioactifs anioniques, non-ioniques, cationiques, ou amphotères.

A titre de tensio-actifs anioniques, on peut citer les alkyléthersulfates, les alkylbenzène sulfonates, les alkylsulfates, les oléfines sulfonates et les alkyléthercarboxylates, salifiés par un ou plusieurs cations. A titre de tensio-actif non ionique, on peut citer les esters d'acides gras de sorbitan, les esters d'acides gras de sorbitan polyoxyéthylé et/ou polyoxypropylé et les alkylpolyglycosides tels ceux cités dans la demande de brevet EP-A-77.167. A titre de tensio-actifs amphotères, on peut citer la bétaine, la sultaïne, l'imidazoline et leurs dérivés. A titre de tensio-actifs cationiques, on peut citer les halogénures d'ammonium quaternaire comme le chlorure de didécylaurylammonium ou les chlorure et bromure de benzylalkonnium.

Par ailleurs, la composition selon l'invention peut comporter :
- des agents classiquement mis en oeuvre pour stabiliser les compositions de peroxyde, tels par exemple les composés phosphoniques organiques, le pyrophosphate acide de sodium, l'acide dipicolinique ou les dérivés de l'étain tel le stannate de sodium,
- de 0,1 % à 1 % d'un acide fort tel l'acide nitrique, l'acide phosphorique, l'acide sulfurique ou l'acide chlorhydrique ces acides sont utiles, de manière connue, en tant qu'agent anti-corrosion et/ou catalyseur de formation dans les compositions contenant des peroxyacides,
- un agent épaississant tel les composés polyoxyéthylénés et polyoxypropylés de poids moléculaire compris entre 1 000 et 20 000, tel ceux commercialisés sous la marque DAPRAL® par la société AKZO,
- des parfums,
- des émollients,
- des adoucissants tels le glycérol.

Une composition selon l'invention peut être préparée par simple mélange de ces constituants. Il peut être avantageux de préparer dans un premier temps une solution de peroxyde non colorée, par exemple selon les procédés décrits dans les demandes de brevets EP-A-24.219, EP-A-193.416 et EP-A-370.850, au nom de L'Air Liquide, puis, dans un deuxième temps, on ajoute à cette solution, l'agent colorant qui peut être soit en poudre, soit en solution ou en dispersion dans l'eau.

Selon un autre aspect, l'invention concerne également l'utilisation des compositions liquides désinfectantes décrites ci-dessus. Ces compositions peuvent être notamment utilisées pour la désinfection et la coloration d'un article ou d'une surface, notamment une surface d'un corps humain ou animal, généralement la peau et les muqueuses, telles par exemple, les trayons de bovins.

La coloration de l'article ou de la surface par la composition selon l'invention permet de marquer et d'identifier les zones traitées et de s'assurer qu'elles ont bien été désinfectées.

La composition selon l'invention peut également être utilisée pour la désinfection des eaux et des canalisations d'eau. Lorsque la composition selon l'invention est diluée dans l'eau à désinfecter, celle-ci se colore légèrement, ce qui permet de suivre son parcours et d'identifier les volumes désinfectés.

Selon un autre aspect encore, l'invention consiste en l'utilisation décrites ci-dessus, pour la coloration et la désinfection ou la stérilisation d'un déchet, en particulier d'un déchet hospitalier jetable, contaminé par des microorganismes tels des virus, des bactéries ou des champignons, tels des levures. Le traitement du déchet peut se faire par simple contact, par exemple par trempage, de celui-ci avec ladite composition.

A ce jour, de tels déchets, souvent à base de matériaux polymères, comme des filtres d'hémodialyse ou de seringues sont habituellement détruits par incinération. Cette incinération est coûteuse et surtout présente des risques pour l'environnement. La désinfection ou la stérilisation de ces articles par des solutions d'agents désinfectants classiques, non colorées, n'a à ce jour, pas été envisagée. En effet, avec de telles solutions, la certitude que l'article a effectivement été désinfecté n'est jamais totale. Il n'est donc pas possible de mettre de tels articles à la décharge sans risque, là encore, pour l'environnement et la santé publique.

Au contraire, lorsque ces articles sont désinfectés ou stérilisés avec des compositions liquides colorées, ils sont alors eux-mêmes colorés; ils peuvent donc être facilement identifiés comme ayant été désinfectés ou stérilisés et mis à la décharge sans risque pour l'environnement.

Les exemples qui suivent ont pour but d'illustrer la présente invention.

Dans tous les exemples, les solutions de peroxyde d'hydrogène mises en oeuvre ont été purifiées par distillation.

### Exemple 1

On a préparé une solution aqueuse par simple mélange sous agitation de ces constituants.

A l'équilibre, la composition de cette solution aqueuse était la suivante (en % en poids) :

| | |
|---|---|
| peroxyde d'hydrogène | 7 % |
| acide acétique | 3,9 % |
| acide peracétique | 0,35 % |
| stabilisant | 0,06 % |
| Orange soleil W 2002 | 0,02 % |
| eau qsp | 100 % |

L'orange soleil W2002 est un agent colorant commercialisé par la société Wackherr, dont la structure chimique est la suivante :

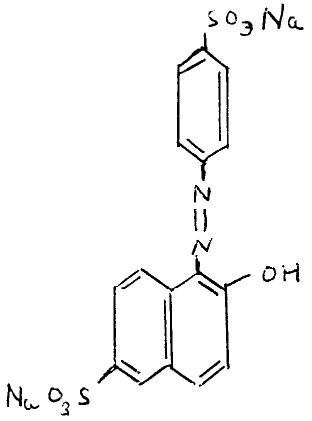

L'intensité chromatique de cette solution aqueuse à l'équilibre était de 73,8. L'intensité chromatique a été déterminée par le rapport entre la mesure de la transmission de lumière dans de l'eau distillée et la mesure de la transmission de lumière dans ladite solution aqueuse. Ces mesures ont été effectuées au moyen d'un chromamètre Minolta CT 310, cuve en verre de 2 mm, Light source D65.

Au bout de deux mois, I'intensité chromatique de la solution aqueuse, mesurée dans les conditions indiquées ci-dessus était de 74,0.

La teneur en peroxydes de la solution aqueuse a été mesurée par cérimétrie. Après deux mois, la teneur en peroxyde d'hydrogène était de 6,98 % et celle en acide peracétique de 0,35 %.

Aux erreurs de mesure près, la coloration et la teneur en peroxydes de la solution aqueuse se sont avérées quasi inchangées après deux mois de stockage.

### Exemple 2

Dans un bécher d'un litre, sous agitation, on introduit les composés suivants, dans l'ordre indiqué :
- eau distillée 912,3 g
- pyrophosphate acide de sodium 0,2 g
- solution à 35 % en poids de peroxyde d'hydrogène 80 g
- acide acétique 5 g
- solution aqueuse à 1 % de Rouge vif W 3002 10 g

A l'équilibre, la teneur en poids en acide peracétique est de 0,01 % et celle en peroxyde d'hydrogène est de 2,8 %.

Après six mois de stockage à l'abri de la lumière et à 20°C, I'intensité colorimétrique et les teneurs en peroxyde d'hydrogène et acide peracétique, mesurées dans les conditions de l'exemple 1, sont restées inchangées.

Le rouge vif W 3002 est un agent colorant commercialisé par la Société Wackher, dont la structure chimique est la suivante:

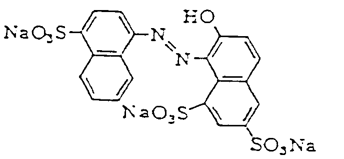

### Exemple 3

Dans un bécher d'un litre, sous agitation, on introduit les composés suivants, dans l'ordre indiqué :
- eau distillée 912,3 g
- pyrophosphate acide de sodium 0,2 g
- solution aqueuse de peroxyde d'hydrogène à 35% en poids 80 g
- acide acétique 5 g
- solution aqueuse à 1 % d'Orange Soleil W 2002 10 g

A l'équilibre, la teneur en poids en acide peracétique était de 0,01% en poids, et celle en peroxyde d'hydrogène de 2,8 %.

Après six mois de stockage à l'abri de la lumière et à 20°C, la coloration et les teneurs en peroxyde d'hydrogène et en acide peracétique de la solution aqueuse, mesurées dans les conditions de l'exemple 1, étaient inchangées.

### Exemple 4

Dans un réacteur de 2 litres, agité mécaniquement, on introduit les composés suivants, dans l'ordre indiqué :
- eau distillée 792,65 g
- solution aqueuse de peroxyde d'hydrogène à 35 % en poids 27,40 g
- acide acétique 3,1 g
- Bactipal™ D(1) 2,7 g
- Dapral T210(2) 35 g
- Oramix NS 10(3) 18,2 g
- Texapon NS0(4) 20,25 g
- Glycérol 100 g
- pyrophosphate acide de sodium 0,2 g
- solution aqueuse à 1% en poids d'orange soleil W2002 10 cm³

(1) solution aqueuse comportant de l'acide peracétique et de peroxyde d'hydrogène commercialisé par la société SEPPIC.
(2) polyalkylèneglycol commercialisé par la société AKZO.
(3) alkylpolyglucoside commercialisé par la société SEPPIC
(4) solution de lauryléthersulfate de Na commercialisée par la société SIDOBRE SINNOVA.

A l'équilibre, la teneur en acide peracétique est de 0,003% en poids et celle en peroxyde d'hydrogène était de 1% en poids.

Sept mois après un stockage à l'abri de la lumière et à 20°C, la coloration et les teneurs en peroxyde d'hydrogène et acide peracétique de la solution aqueuse, mesurées dans les conditions de l'exemple 1, étaient inchangées.

### Exemple 6 :

On a préparé par simple mélange de ses constituants, une solution aqueuse dont la composition à l'équilibre est la suivante (en poids) :
- acide peracétique 0,00095 %
- acide acétique 0,05 %
- peroxyde d'hydrogène 2,8 %
- HOSTAFINE Bleu B26(1) 0,01 %
- eau, q.s.p. 100 %

(1) Dispersion de pigment de phtalocyanine commercialisée par la société Hoechst.

L'intensité chromatique a été mesurée dans les conditions de l'exemple 1 mais avec une cuve de 1 cm.

Au bout de 7 mois et demi, la variation de l'intensité chromatique était inférieure à 2 %. Autrement dit, la varation de la coloration de cette solution n'était pas visible à l'oeil.

### Exemple 7 :

On a reproduit l'exemple 6 en remplaçant l'agent colorant par de l'HOSTAFINE vert GN (dispersion de pigment de phtalocyanine commercialisée par la société Hoechst).

La variation de l'intensité chromatique, mesurée dans les conditions de l'exemple 5, était inférieure à 2 % après 7 mois et demi.

### Exemple 8 :

On a reproduit l'exemple 6 en remplaçant l'agent colorant par 0,01 % d'un colorant acide E102 conforme à l'invention, de formule suivante :

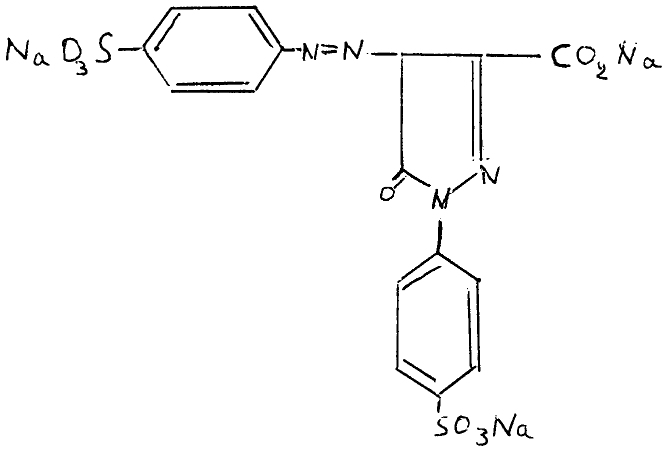

La variation de l'intensité chromatique, mesurée dans les conditions de l'exemple 5, était inférieure à 2 % après 7 mois et demi.

### Exemple 9 (comparatif) :

On a reproduit l'exemple 6 en remplaçant l'agent colorant par 0,01 % d'un agent colorant décrit par H. Mücke, Pharmazie, 29, H.3 (1974), pp. 206-207, à savoir le bleu de méthylène. Seize heures après avoir atteint l'équilibre de la solution, son intensité chromatique avait varié de plus de 10 %.

Ainsi, la couleur bleu violet initiale de la solution s'était déplacée vers une couleur bleu vert.

## Revendications

1. Composition liquide désinfectante caractérisée en ce qu'elle comporte au moins un peroxyacide désinfectant et au moins un agent colorant, ledit peroxyacide organique étant choisi dans le groupe constitué par :
1) un monoperoxyacide de formule (I)
R₁ - CO₃H (I)
dans laquelle R₁ représente un radical alkyle en C₁ - C₂₄, linéaire ou ramifié, un radical aryle ou un radical cycloalkyle en C₃ - C₁₀.
2) un diperoxyacide de formule (II) :
H₃OC - R₂ - CO₃H (II)
dans laquelle R₂ représente un radical alkyle en C₁ - C₂₄ linéaire ou ramifié, un radical arylène ou un radical cycloalkylène en C₃ - C₁₀,
chacun de R₁ ou de R₂ étant substitué ou non par un ou plusieurs groupes fonctionnels ou radicaux, ladite composition étant stable pendant plus de quinze jours.

2. Composition selon la revendication 1, caractérisée en ce que le peroxyacide organique est un composé de formule (I) où R₁ est un radical alkyle en C₁ - C₁₂.

3. Composition selon la revendication 2, caractérisée en ce que le peroxyacide organique est l'acide peracétique.

4. Composition selon l'une des revendications 1 à 3, caractérisée en ce qu'elle comporte de 0,0001 % à 5 % en poids, de préférence de 0,001 % à 0,4 % en poids de peroxyacide organique.

5. Composition selon l'une des revendications 1 à 4, caractérisée en ce qu'elle comporte de 0,001 % à 35 % en poids, de préférence de 0,1% à 10% en poids de peroxyde d'hydrogène.

6. Composition selon l'une des revendications 1 à 5, caractérisée en ce que l'agent colorant est un composé monoazo.

7. Composition selon la revendication 6, caractérisée en ce que l'agent colorant est un composé monoazo de formule (III) :
R₃ -N=N-R₄ (III)
dans laquelle R₃ et R₄, identiques ou différents, représentent un radical aryle, de préférence un radical naphtyle ou un radical phényle, chacun de R₃ et R₄ étant substitué ou non par un à trois groupes fonctionnels OH ou -SO₃M, M étant l'hydrogène ou un cation d'un métal alcalin tel le sodium.

8. Composition selon l'une des revendications 1 à 5, caractérisée en ce que l'agent colorant est un pigment.

9. Composition selon la revendication 7, caractérisée en ce que le pigment est une phtalocyanine ou une dispersion comprenant une phtalocyanine.

10. Composition selon l'une des revendications 1 à 9, caractérisée en ce qu'elle comporte de 0,0001 % à 1 % en poids, de préférence de 0,001 % à 0,5 % en poids dudit agent colorant.

11. Composition liquide désinfectante caractérisée en ce qu'elle consiste en une solution aqueuse comprenant 0,1 % à 10 % en poids de peroxyde d'hydrogène, de 0,001 % à 0,4 % en poids d'un peroxyacide organique, de 0,1 % à 10 % en poids d'un acide carboxylique et de 0,0001% à 0,03 % en poids d'un agent colorant consistant en un composé azo et/ou une phtalocyanine et de l'eau.

12. Composition selon la revendication 11, caractérisée en ce que le peroxyacide organique est un composé de formule (I) ou de formule (II) telles que définies dans la revendication 1, l'acide carboxylique étant un composé, respectivement, de formule (IV)
R₁COOH (IV)
ou de formule (V)
HOOC-R₂-COOH (V)
R₁ et R₂ ayant la même signification que celle dudit peroxyacide organique.

13. Composition selon la revendication 12, caractérisée en ce que le peroxyacide organique est l'acide péracétique et l'acide carboxylique est l'acide acétique.

14. Utilisation d'une composition selon l'une des revendications 1 à 13 pour la désinfection et la coloration d'un article ou d'une surface.

15. Utilisation selon la revendication 13, caractérisée en ce que ladite surface est celle d'un corps humain ou animal, comme la peau ou les muqueuses.

16. Utilisation d'une composition selon l'une des revendications 1 à 13 pour la désinfection des eaux et des canalisations d'eau.

17. Utilisation d'une composition selon l'une des revendications 1 à 13, pour la désinfection d'un déchet, tel un déchet hospitalier.
